# EUROPEAN PATENT SPECIFICATION

(11) **EP 0 537 161 B1**
(45) Date of publication and mention of the grant of the patent: **02.10.1996**
(21) Application number: 91908531.6
(22) Date of filing: 19.04.1991
(51) Int. Cl.: A61B 5/00

(54) **ALARM SYSTEM**
ALARMSYSTEM
SYSTEME D'ALARME

(30) Priority: 21.04.1990 GB 9008992
(43) Date of publication of application: 21.04.1993
(73) Proprietor: KINGSTON, John Edward, South Humberside DN33 3DX (GB)
(72) Inventor: KINGSTON, John Edward, South Humberside DN33 3DX (GB)
(74) Representative: Craske, Stephen Allan
(86) International application number: GB9100620
(87) International publication number: WO9115989

(56) References cited:
- DE-A- 2 632 899
- FR-A- 2 425 230
- GB-A- 1 543 441
- US-A- 4 819 860

## Description

### TECHNICAL FIELD OF THE INVENTION

This invention relates to personal alarm systems for indicating when a user is in distress or requires assistance.

### BACKGROUND ART

GB 810 730 discloses a small radio transmitter which is worn on the wrist and transmits an audio signal to reveal the wearer's position if he/she is accidentally buried. In todays crowded urban environment however, the transmission of an audio signal alone would result in a high risk of false alarms.

It is also known from GB 1 543 441, US 4 510 350 and US 4 819 860 to provide a wrist mounted transmitter which transmits a digital identification code. In the case of US 4 510 350 the transmitter is triggered to send a pre-recorded digitised vocal message over the telephone network by simultaneous depression of two buttons. FR-A-2 425 235 also proposes a miniature transmitter which generates predetermined codes.

Whilst the transmission of an encoded signal would reduce or eliminate the risk of false alarms, the present invention is based on the premise that many people would find it more reassuring to know that their cries of help would be heard if they should come face to face with an aggressor for example. In addition, it is believed that many people would tend to react quicker to cries of help than they would to an alarm bell or siren.

### SUMMARY OF THE INVENTION

The present invention proposes that, when activated, a personal alarm signalling device transmits audio signals picked up by a microphone together with an encoded identification signal.

To ensure efficient identification of the signalling device the identification signal is preferably repeated numerous times before the audio signals are transmitted.

The identification signal may initiate an audible and/or visual alarm which alternates with the public broadcasting of the audio signals from the microphone. Alternatively, the identification signal may initiate a telephone call to a monitoring base to which the audio signals are then sent.

The signalling device preferably receives the identification code from the control means, into which the code may be programmed by the user.

### BRIEF DESCRIPTION OF THE DRAWINGS

The invention is exemplified in the accompanying drawings, in which:
Figure 1 is a front view of an alarm signalling device for use an alarm system in the invention,
Figure 2 is a block circuit diagram of the signalling device,
Figure 3 is a general view of a control box and alarm box for use with the signalling device, and
Figure 4 is a block circuit diagram of the alarm and control boxes.

### DETAILED DESCRIPTION OF THE DRAWINGS

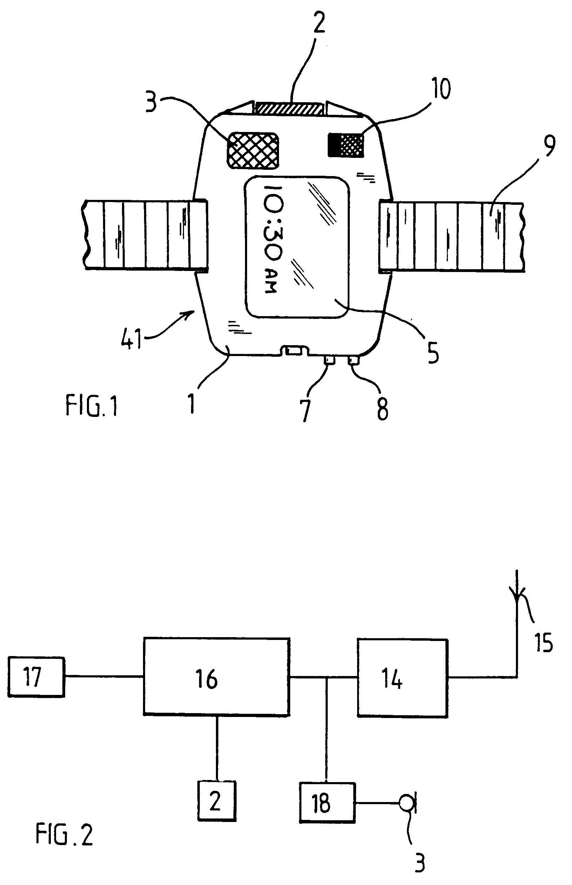

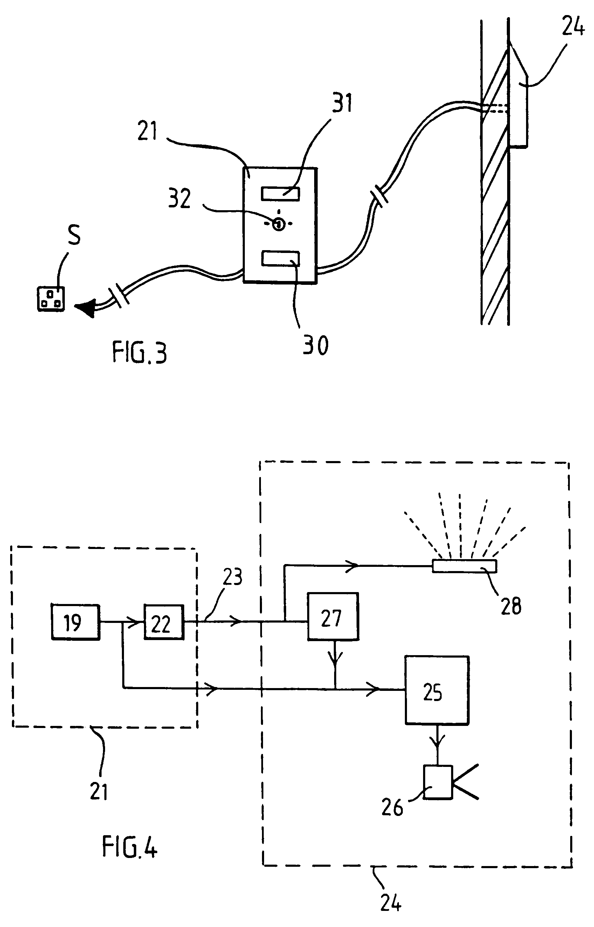

The alarm system includes a signalling device 41 (Fig. 1), a control box 21 (Fig. 3) including a cradle 30 for receiving the signalling device, and an alarm box 24.

Referring to Fig 1, the signalling device 41 comprises a housing 1 of durable plastics having an emergency button 2 mounted in its wall. The front face of the housing includes a microphone 3 and a liquid crystal display panel 5 which can give a time readout as in a digital watch. On the side of the housing there are two "set" buttons 7 and 8. The housing is secured to a wrist band 9, but the housing may also be adapted to be worn anywhere on the body, for example suspended on a loop worn around the neck, on a belt or key ring, or to be attached to the clothing by safety pins, a spring clip, or more secure means. The display panel 5 and buttons 7 and 8 could be omitted if desired.

Referring now to Fig. 2, the housing contains a micro-transmitter 14 which generates a low power radio signal in the 49 MHz region, although other frequencies could be used. The signal is transmitted from a wire antenna 15 which can be incorporated into the wrist band 9, or the loop if worn around the neck, or internally within the housing. The transmitter can be powered from internal rechargeable batteries (not shown) which can be re-charged by conventional means or, by placing the signalling device in the cradle 30, the batteries can be re-charged from the control box 21 via suitable electrical contacts provided on the signalling device and the cradle. The transmitter could also be powered by non-rechargeable batteries if required. An audio and/or visual battery condition indicator 10 (Fig. 1) may be provided on the signalling device to inform the user when the batteries are low and require recharging or replacing.

Referring back to Fig. 2, a frequency shift keying device 16 is arranged to modulate the transmitter with a binary identification code held in shift registers 17. The method and type of coding depends upon the level of security demanded of the system. The code may be set by the user by means of the buttons 7 and 8, which can also be used for setting the watch, or the code could be automatically transferred from the control box into the shift registers when the signalling device is placed in the cradle 30. The code could also he set internally via miniature switches or links on the printed circuit board during manufacture. In addition to an identification code, further codes could be transmitted, for example to enable a user to signal which of the emergency services is required, e.g. fire, police or ambulance, via the public telephone system. The transmitter can also be phase modulated by the audio signals from the microphone 3 via an automatic gain control circuit 18.

Referring now to Fig.s 3 and 4, the signals sent out by the signalling device 41 are picked up by a receiver 19 mounted in the control box 21. The control box may be mounted in a convenient location inside the building, powered either direct from the mains or via a mains socket S, with the back-up of rechargeable batteries in the event of a mains power failure. The receiver 19 demodulates the audio signals received from the transmitter 14 and also sends the coded signals which it receives to a code recognition circuit 22. Recognition of an appropriately coded signal results in the generation of an alarm signal at 23. The control box is hard-wired to an alarm box 24 mounted on the outside of a building. The alarm box contains rechargeable batteries (not shown) which are powered from the control box 21 , together with an audio amplifier 25 which feeds a loudspeaker 26. The audio amplifier 25 is driven by an audio tone generator 27 which is activated by the alarm signal from the control box. The alarm signal also activates a xenon flasher tube 28 mounted in the alarm box. The amplifier also receives the demodulated audio signals.

As well as allowing for re-charging the signalling device when rechargeable batteries are used, the signalling device can be programmed with the identification code from the control box, as noted above. The code can be entered into the control box by the user by means of a set of external miniature switches or a keypad 31. The code is automatically transferred to the shift registers of the signalling device each time the device is placed in the cradle 30. This allows the user to set his own identification code. Alternatively, the code can be pre-programmed into the control box at point of manufacture by internal miniature buttons, links or switches mounted on an internal printed circuit board. In this case the same code must be pre-programmed into the signalling device at point of manufacture, and it will not therefore be necessary to transfer the code via the cradle.

The control box may also incorporate a 3-digit, 7 segment led display 31. A keypad or three position key switch 32 is provided on the control box 21 to place the system into an ARM, RESET or TEST mode. In the ARM mode the display 31 could show the message SET to indicate that the system has been set ready for use, and in the RESET mode the message "rES" is displayed to show that the system is in a reset state, i.e. inactive. When the system is in the TEST mode the digital display on the control box indicates "tES". If the emergency button 2 is now depressed the display will change to indicate the decimal value of the transmitted identification code, but the alarm box is not activated. This is useful in setting up the system and in fault finding. The test mode is terminated by selecting one of the other two modes. To test the alarm box the emergency button 2 should be depressed whilst the system is in the normal ARM mode, causing the system to be fully activated, as will now be described.

When the user depresses the alarm button 2 the transmitter 14 sends the coded signal for a period of approximately five seconds. During this time the coded signal is repeated some hundred or so times to reduce recognition errors. When the recognition circuit 22 responds to the code, it sends an alarm signal to the alarm box which activates the tone generator 27 so that a warning tone is emitted by the loudspeaker 26 for about seven seconds, and the xenon tube 28 also gives out a visual indication. The transmitter then switches to a period of transmitting voice signals from the microphone 3. The control box receives these signals and sends them to the alarm box 24 to be broadcast via the loudspeaker to the outside world.

If the control box detects silence for a period of fifteen seconds the audible alarm is generated for a further seven seconds. This sequence is repeated until the signalling device is placed in the cradle 22 and the keypad or key switch is placed in the "RESET" mode, which causes the transmitter and the alarm box to be de-activated.

Multiple slave alarm boxes and repeaters can be attached to one control box enabling the coded signal and voice transmissions to be received and broadcast over a large area.

The system could be interfaced to the public telephone network via the control panel or the alarm box or by incorporating the receiver unit into a telephone console. Reception of the appropriate identification code could cause the console to generate selected preset or user-programmed telephone numbers. Thus, when the button 2 is depressed a designated monitoring base can monitor voice transmissions directly from the user, enabling the base to initiate the appropriate emergency action with minimum time delay.

In order that the system may be interfaced to existing security systems a relay output (not shown) is provided. The system is also designed to interface with compatible accessories. The control box can incorporate a standard cassette tape recorder for recording the audio signals to help trace and convict aggressors for example.

The equipment described could for example be installed in the home of an elderly person. The reproduction of audio signals by the external speaker will alert neighbours or passers by that the occupier requires assistance. This acts to reassure the user that if they should come face to face with an aggressor their cries of help will be heard. People also tend to react quicker to cries of help than they do to an alarm bell or siren.

By using a number of signalling units with different identification codes a number of individuals can be simultaneously monitored, in a nursing home or hospital for example. A separate monitoring unit could be sited on each floor of a residential nursing home, the unit having a panel with audible and/or visual indicators to indicate where each of the monitored people is located on that particular floor. A portable hand held receiver and alarm could be used instead of the fixed alarm and control box.

## Claims

1. An alarm system which includes an alarm signalling device comprising a housing (1) adapted to be worn on the person, the alarm signalling device including transmitter means (14) for sending out radio signals,
characterised in that the alarm signalling device further includes a microphone (3) for picking up sounds and producing audio signals to modulate said transmitter means, emergency trigger means (2) for operating the transmitter means (14), and code generator means (16) for modulating the transmitter means with an encoded identification signal, said transmitter means, said microphone, said trigger means and said code generator means being operatively interconnected, and said transmitter means (14) being constructed to transmit, for an initial period, encoded information from said code generator means (16), and to thereafter switch automatically to transmit said audio signals for a second period.

2. An alarm system according to Claim 1, in which said transmitter means (14) is constructed to repeatedly transmit the encoded identification signal during said initial period.

3. An alarm system according to Claim 1 or 2, including control means (21) which comprises radio receiver means (19) for receiving and demodulating said radio signals sent out by said transmitter means (14), and a recognition circuit (22) for generating an alarm signal when the correct encoded identification signal is received, the alarm signal being arranged to operate audible (26) and/or visual (28) alarm means, and the receiver means (19) being arranged to send said audio signals to loudspeaker means (26).

4. An alarm system according to Claim 3, in which said control means is constructed to operate the alarm means for periods which alternate with intermediate periods in which said audio signals drive the loudspeaker means (26).

5. An alarm system according to Claim 1 or 2, including control means which comprises radio receiver means and a recognition circuit for initiating a telephone call to a remote location via a telephone system when the correct encoded identification signal is received, the control means being arranged to feed said audio signals to the remote location via the telephone system.

6. An alarm system according to Claim 1 or 2, including control means (21) for storing an identification code and including connection means (30) for transferring the identification code to the signalling device, said signalling device including store means (17) for storing the received identification code.

7. An alarm system according to Claim 6, in which the control means (21) is arranged to permit the user to enter the identification code into the control means.

## Patentansprüche

1. Alarmapparat, der eine Alarmsignalisierungsvorrichtung enthält und ein Gehäuse (1) umfasst, das dazu ausgelegt ist, auf dem Benutzer selbst getragen zu werden, wobei die Alarmsignalisierungsvorrichtung ein Sendemittel (14 zur Aussendung von Radiosignalen einschliesst,
dadurch gekennzeichnet, dass die Alarmsignalisierungsvorrichtung ausserdem ein Mikrophon (3) zum Auffangen von Schall bzw. zur Herstellung von Hörsignalen zur Modulierung des erwähnten Sendemittels, ein Notauslösemittel (2) zur Betätigung des Sendmittels (14) sowie ein Codeherstellungsmittel (16) zur Modulierung des Sendemittels mit einem verschlüsselten Identifizierungssignal umfasst, wobei das erwähnte Sendemittel, das erwähnte Mikrophon, das erwähnte Auslösemittel und das erwähnte Codeherstellungsmittel betrieblich untereinander verbunden sind und das erwähnte Sendemittel (14) dazu ausgelegt ist, während einer Anfangsperiode verschlüsselte Informationen vom erwähnten Codeherstellungsmittel zu übertragen und danach automatisch umzuschalten,um die erwähnten Hörsignale während einer zweiten Periode zu übertragen.

2. Alarmapparat nach Patentanspruch 1, bei dem das erwähnte Sendemittel dazu ausgelegt ist, während der erwähnten Anfangsperiode das verschlüsselte Identifizierungssignal wiederholt zu übertragen.

3. Alarmapparat nach Patentanspruch 1 oder 2, der eine Steuereinrichtung,
die ein Radioempfangsmittel (19) zum Empfang bzw.zur Demodulierung der erwähnten, durch das erwähnte Sendemittel (14) ausgesandten Radiosignale, sowie einen Erkennungsschaltkreis (22) einschliesst, der zur Herstellung eines Alarmsignals beim Empfang des richtigen verschlüsselten Identifizierungssignals dient, wobei das Alarmsignal dazu ausgelegt ist, hörbare (26) und / oder sichtbare (28) Alarmvorrichtungen zu betätigen, und das Empfangsmittel (19) dazuausgelegt ist, Hörsignale an eine Lautsprechereinrichtung zu übermitteln.

4. Alarmapparat nach Patentanspruch 3, bei dem die erwähnte Steuereinrichtung dazu ausgelegt ist, die Alarmeinrichtung während Perioden zu betätigen, die mit Zwischenperioden alternieren, während derer die Lautspecher einrichtung (26) erwähnten Hörsignalen betätigt wird.

5. Alarmapparat nach Patentanspruch 1 oder 2, der eine Steuereinrichtung einschliesst, die ein Radioempfangsmittel sowie einen Erkennungsschaltkreis umfasst, die dazu dient, über eine Telefonanlage einen Telefonanruf an eine entfernte Stelle zu bewirken, wenn das richtige verschlüsselte Identifizierungssignal empfangen wird, wobei die Steuereinrichtung dazu ausgelegt ist, die erwähnten Hörsignale über die Telefonanlage an die entfernte Stelle su übermitteln.

6. Alarmapparat nach Patentanspruch 1 oder 2,der eine Steuereinrichtung (21) zur Speicherung eines Identifizierungscodes sowie ein Verbindungsmittel (30) zur Übertragung des Identifizierungscodes an die Signalisierungsvorrichtung umfasst, wobei die erwähnte Signalisierungsvorrichtung eine Speichereinrichtung zur Speicherung des empfangenen Identifizierungscodes einschliesst.

7. Alarmapparat nach Patentanspruch 6, bei dem die Steuereinrichtung (21) dazu ausgelegt ist, es dem Benutzer zu ermöglichen, den Identifizierungscode in die Steuereinrichtung einzugeben.

## Revendications

1. Système d'alarme pourvu d'un dispositif der signalisation d'alarme comprenant un logement (1) adapté à être porté sur l'utilisateur, ledit dispositif de signalisation d'alarme comprenant des moyens de transmission (14) servant à émettre des signaux radiophoniques,
caractérisé en ce que le dispositif de signalisation d'alarme est également pourvu d'un microphone (3) servant à capter des sons et à générer des signaux sonores afin de moduler lesdits moyens de transmission, de moyens d'amorçage de secours (2) servant à actionner lesdits moyens de transmission (14) ainsi que de moyens générateurs de code (16) servant à moduler lesdits moyens de transmission avec un signal d'identification encrypté, lesdits moyens de transmission, ledit microphone, lesdits moyens d'amorçage et lesdits moyens générateurs de code étant fonctionnellement interconnectés et lesdits moyens de transmission (14) étant construits pour transmettre pendant un délai initial des informations encryptées à partir desdits moyens générateurs de code (16) et pour commuter ensuite automatiquement afin de transmettre lesdits signaux sonores pendant un deuxième délai.

2. Système d'alarme selon la Rev.1, dans lequel les moyens de transmission (14) sont construits pour transmettre à plusieurs reprises au cours dudit délai initial le signal d'identification encrypté.

3. Système d'alarme selon la Rev. 1 ou 2, pourvu de moyens de commande (21) qui comprennent des moyens de radioréception (19) servant à recevoir et à démoduler lesdits signaux radiophoniques émis par lesdits moyens de transmission (14), ainsi que d'un circuit de reconnaissance (22) servant à générer un signal d'alarme lorsque le signal d'identification encrypté correct est reçu, le signal d'alarme étant adapté pour actionner des moyens d'alarme sonores (26) et/ou visuels (28) et lesdits moyens de réception (19) étant adaptés pour émettre lesdits signaux sonores vers des moyens de réception amplifiées.

4. Système d'alarme selon la Rev. 3, dans lequel lesdits moyens de commande sont construits pour actionner les moyens d'alarme pendant des délais qui alternent avec des délai intermédiaires pendant lesquels lesdits signaux sonores actionnent les moyens de réception amplifiées.

5. Système d'alarme selon la Rev. 1 ou 2, pourvu de moyens de commande comprenant des moyens de radio réception ainsi qu'un circuit de reconnaissance servant à initier un appel téléphonique vers un poste à distance au moyen d'un système teléphonique lorsque le signal d'identification encrypté correct est reçu, les moyens de commande étant construits pour transmettre lesdits signaux sonores au poste à distance au moyen du système téléphonique.

6. Système d'alarme selon la Rev. 1 ou 2, comprenant des moyens de commande (21) servant à mémoriser un code d'identification et comprenant également des moyens de connection (30) servant à transférer le code d'identification vers le dispositif de signalisation, ledit dispositif de signalisation comprenant des moyens de mémorisation (17) servnat à mémoriser le code d'identification reçu.

7. Système d'alarme selon la Rev. 6, dans lequel les moyens de commande (21) sont construits de manière à permettre à l'utilisateur d'inscrire le code d'identification dans lesdits moyens de commande.
